# EUROPEAN PATENT APPLICATION

(11) **EP 2 332 602 A1**
(43) Date of publication of application: **15.06.2011**
(21) Application number: 09405220.6
(22) Date of filing: 09.12.2009
(51) Int. Cl.: A61M 5/315

(54) **Container for receiving liquids**

(71) Applicant: F. Hoffmann-La Roche AG, 4070 Basel (CH); Roche Diagnostics GmbH, 68305 Mannheim (DE)
(72) Inventor: McLaren, James, Grafham Cambridgeshire PE28 0BD (GB); Pearson, Allen, Wistow Cambridgeshire PE28 2QB (GB); Hänggi, Roger, 4208 Nunningen (CH)
(74) Representative: Münch, Martin Walter

(57) **Abstract**

The invention concerns a container for receiving a liquid drug, comprising a container body (1) with a cylindrical interior space and with a piston (2) arranged therein, which in liquid-tight manner abuts to the boundary walls of interior space and by doing so is movable within said interior space in order to increase or decrease the volume of the container. The piston (2) provides coupling means (4) for coupling a piston rod (5) in a detachable manner to it for moving it forward and backward. These coupling means (4) interact with the container body (1) in such a manner that, between a fully retracted position of the piston (2) and all other positions of the piston (2) they change their shape.

By means of this design it becomes possible to provide a syringe-type container having a piston (2) which can be moved forward and backward by means of a dedicated piston rod (5) but cannot be removed from the container body (1) by means of this piston rod (5) even in case there is no physical stop for the piston (2) at the end of the container body (1).

## Description

The present invention relates to a container for receiving liquids, to an arrangement comprising the container and to a method of operating the arrangement according to the preambles of the independent claims.

A typical problem when filling cylinder-piston configurations, like e.g. ampoules or syringe assemblies, with a liquid by retracting the piston inside the cylinder is to make sure that on the one hand the design stroke of the piston can fully be utilized while on the other hand there is no risk that the piston is drawn out of the cylinder body.

A very simple way to solve this problem is to provide a physical stop for the piston at the end of the cylinder, which requires, however, additional operating steps during the manufacturing and/or assembly and thus is undesirable for inexpensive single use articles, like self filled insulin cartridges for the therapy of diabetes.

Hence, it is the object of the invention to provide a syringe-type container for receiving a liquid as well as an arrangement comprising this container in which, even without a physical stop for the piston protruding inwardly at the end of the container body, it can be ensured that the piston cannot be retracted out of the cylinder body of the container.

This object is achieved by the container and the arrangement according to the independent claims.

A first aspect of the invention concerns a container for receiving a liquid, preferably for receiving a liquid drug, like e.g. a liquid pain killer or insulin. The container comprises a container body with a generalized cylindrical interior space, such as a right circular, right rectangular or right elliptical interior space defining a longitudinal axis. Inside the interior space, there is arranged a piston, which in liquid-tight manner abuts to the boundary walls of interior space and by doing so is movable within said interior space along said longitudinal axis of the cylindrical interior space of the container body in order to increase or decrease the volume of a fluid-tight space defined by the boundary walls of interior space and by the front face of the piston.

At its back side, the piston provides coupling means for coupling a piston rod in a detachable manner to the piston in order to be able to move the piston forward and backward within the interior space of the container body by means of the piston rod.

These coupling means of the piston interact with the container body in such a manner that, between a fully retracted position of the piston and all other positions of the piston within said interior space of the container body, they change their shape. In other words, the coupling means of the piston in the fully retracted position of the piston have a different shape than in the rest the positions in which the piston can be positioned within the interior space.

By means of this design it becomes possible to effect a change in the coupling quality with coupling means of a piston rod to be coupled to them between the before mentioned piston positions, namely from a positive or non-positive locking only in pushing forward direction of the piston in the fully retracted position to a positive or non-positive locking in pushing forward direction and in retracting direction of the piston in all other positions of the piston within said interior space of the container body. This in turn makes it possible to provide a syringe-type container for receiving a liquid, the piston of which can be moved forward and backward inside its container body by means of a dedicated piston rod but cannot be removed from the container body by means of this piston rod even if there is no physical stop for the piston directed inward at the end of the container body.

The fully retracted position of the piston is to be understood as a position where the fluid-tight space defined by the boundaries of the interior space and by the front face of the piston has its design volume, i.e. has the maximum volume that has been intended by its design.

In a preferred embodiment of the container, the coupling means of the piston form an axial cavity for receiving a coupling element of the piston rod to be coupled thereto, which cavity in all positions of the piston within the interior space of the container body except in the fully retracted position features one or several undercuts which are shortened or removed when the piston is moved into the fully retracted position within said interior space of the container body.

In a further preferred embodiment of the container, the coupling means of the piston form an axial protrusion for being received in a cavity of an coupling element of a piston rod to be coupled thereto, which protrusion in all positions of the piston except the fully retracted position features one or several undercuts which undercuts are shortened or removed when the piston is moved into the fully retracted position within said interior space of the container body.

These two preferred embodiments of the container allow establishment and termination of a positive or non-positive locking in retracting direction of the coupling means of the piston and the dedicated coupling means of a piston rod to be coupled thereto upon changing between the fully retracted and all other piston positions within said interior space of the container body.

In still a further preferred embodiment of the container, the coupling means of the piston form at least one axial or radial protrusion, which in all positions of the piston except the fully retracted position is positioned in a first axial or radial position and when the piston is moved into the fully retracted position, is moved into a second axial or radial position. By means of this it becomes possible to design the coupling means of a piston rod to be coupled to the piston in such a manner that their shape can be changed in the coupled state by moving the at least one axial or radial protrusion between said first and second axial or radial position and to thereby establish and terminate a positive or non-positive locking in retracting direction of the piston between the coupling means of a piston rod and the coupling means of the piston upon changing between these piston positions within said interior space of the container body.

In still a further preferred embodiment of the invention, the coupling means of the piston comprise one or several actuating elements which in the fully retracted position of the piston radially expand and thereby effect the change in shape of the coupling means.

If, in case of the before mentioned embodiment of the container, the boundary walls of the interior space of the container body comprise one or several recesses or apertures, into which the actuating elements expand when the piston is moved into the fully retracted position within said interior space of the container body, these recesses or apertures in combination with the expanded actuating elements can form a physical stop for the piston which further increases the safety against a pulling of the piston out of the container body.

If, in case of the before mentioned embodiment of the container, the cylindrical interior space of the container body at its back end opens to the outside in such a manner that the piston when travelling backwards theoretically can leave the interior space, and the actuating elements axially move out of the cylindrical interior space and radially expand when the piston is moved into the fully retracted position, it becomes possible e.g. to terminate a positive or non-positive locking in retracting direction of the coupling means of the piston with dedicated coupling means of a piston rod when the back end of the piston starts leaving the interior space of the container body but well before the piston as such is pulled out of the container body. By this, the fully retracted position can be the position when the back end of the piston starts leaving the interior space so that a maximum piston stroke and therewith a maximum design volume of the container results.

Preferably, the actuating elements comprise one or several lugs, which radially expand when the piston is brought into the fully retracted position.

Preferably, the coupling means of the piston are made in one-piece with the body of the piston. More preferably, the entire piston including sealing elements and coupling means is made in one-piece design. By means of this, the manufacturing costs can considerably be reduced compared to containers with pistons made in multi-piece design.

In yet another preferred embodiment, the container is a sterile container for medical use, preferably with a maximum volume with the piston arranged in the fully retracted position of less than 50 ml, preferably of less than 10 ml. In that case it is furthermore preferred that at the front end the container has a septum which can be pierced in order to get access to its fluid-tight inner space defined by the boundary walls of interior space and by the front face of the piston. Such embodiments of the container are preferred commercial products.

A second aspect of the invention concerns an arrangement comprising at least a container according to the first aspect of the invention and a piston rod for moving the piston of the container forward and backward within the interior space of the container body. The piston rod comprises coupling means which are especially adapted to interact in the coupled state with the coupling means of the piston. This interaction is such that when the piston is not in the fully retracted position, there exists a positive or non-positive locking between the piston and the piston rod in pushing forward direction as well as in retracting direction of the piston and that when the piston is retracted by means of the piston rod into the fully retracted position, the positive or non-positive locking between the piston and the piston rod in retracting direction is terminated. In that state, upon a further moving of the piston rod in the retraction direction, the piston rod automatically separates from the piston and even without a physical stop for the piston there is no risk that the piston is further pulled in retracting direction which would result in the piston being positioned beyond its fully retracted position as defined in here earlier or even in the piston being pulled out of the container body.

In a preferred embodiment of the arrangement, the piston rod is part of an apparatus with drive means for moving the piston rod forward and backward. By means of this, the arrangement is suitable for an automated filling of the container according to the first aspect of the invention by using the first preferred embodiment of the method according to the third aspect of the invention which will be described below.

A third aspect of the invention concerns a method of operating the arrangement according to the second aspect of the invention. The method comprises the steps:
a) connecting the piston rod and the piston of the container by coupling the coupling means of the piston rod and the coupling means of the piston with each other;
b) pushing the piston of the container with the aid of the piston rod forward inside the interior space of the container body, thereby displacing air out of the fluid-tight space of the container defined by the boundary walls of interior space and by the front face of the piston;
c) retracting the piston by means of the piston rod inside the interior space of the container body, thereby receiving a liquid drug, in particular insulin, in the fluid-tight space of the container defined by the boundary walls of interior space and by the front face of the piston; and
d) separating the piston and the piston rod by retracting the piston with the aid of the piston rod into the fully retracted position and by subsequently moving the piston rod further in retracting direction.

This method constitutes the intended use of the arrangement according to the second aspect of the invention.

In a first preferred embodiment of the method, the steps b) and c) are successively performed more than once, thereby stepwise increasing the amount of liquid drug contained inside the fluid-tight space of the container. In particular in cases where the container is an ampoule which is filled with a liquid drug via a cannula, this embodiment provides the advantage that filling of the container can be accomplished in several small steps which helps avoiding the formation of foam and bubbles within the liquid drug filled into the container.

In a further preferred embodiment of the method, the steps b) and c) are successively performed until the fluid-tight space of the container with the piston arranged in the fully retracted position is substantially filled with the liquid drug. By means of this, the design volume of the container can fully be utilized.

Further preferred embodiments of the invention become apparent from the dependent claims and from the following description by way of the drawings. Therein show:
Figures 1a to 1c a longitudinal section through the back end of a container according to the invention coupled with a dedicated piston rod in different situations;
Fig. 1d a longitudinal section through the back end of the container shown in the Figures 1a to 1c with the piston rod separated from the container; and
Fig. 2 a view onto the back end of the container shown in the Figures 1a to 1c.

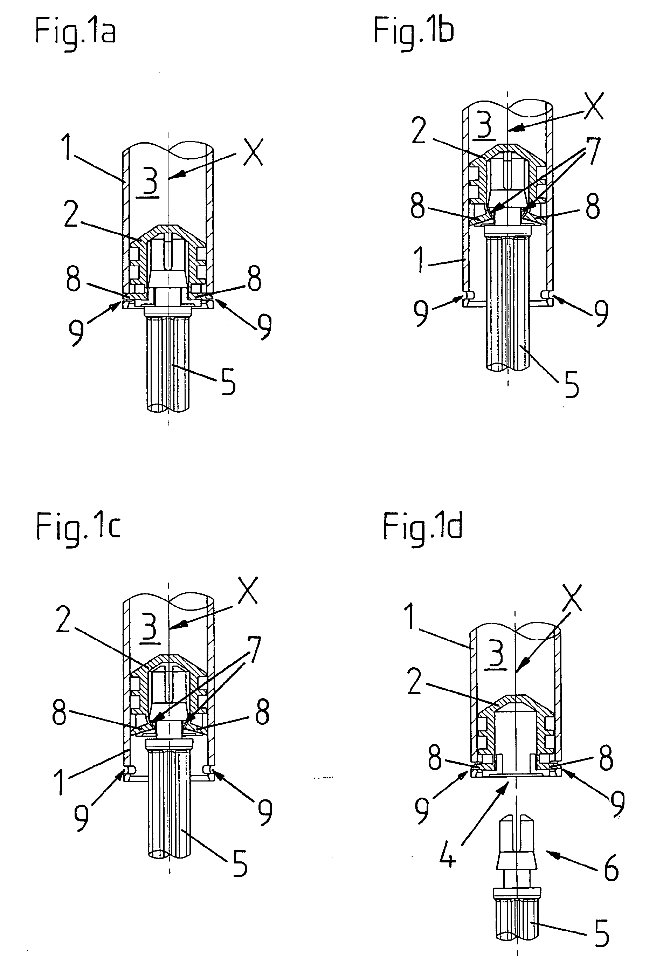

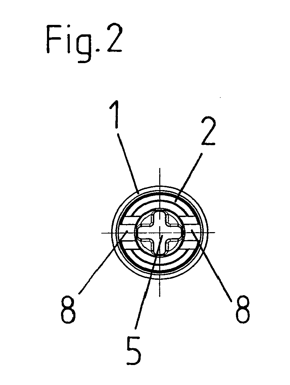

The back end of a container according to the invention, which is formed by a self filling ampoule for use in an insulin pump, is in longitudinal section shown in the Figures 1a to 1d, three times in different states with a dedicated piston rod 5 coupled to the back end of the piston 2 of the container (Figures 1a to 1c) and one time with the piston rod 5 separated from the piston 2 of the container (Fig. 1d). The shown combination of the ampoule according to the first aspect of the invention and a dedicated piston rod 5 forms an arrangement according to the second aspect of the invention. Fig. 2 shows a back end view of the ampoule with the piston rod 5 coupled to the piston 2.

As can be seen from these Figures, the ampoule comprises a container body 1 made of lucent plastics with a circular-cylindrical interior space defining a longitudinal axis X. Within the interior space of the container body 1 there is arranged a piston 2 made from plastics, which in a liquid-tight manner abuts to the boundary walls of interior space and by doing so is movable within the interior space along the longitudinal axis X thereof. The boundary walls of the interior space and the front face of the piston 2 define a fluid-tight space 3, the volume of which can be increased or decreased by moving the piston 2 within the interior space of the container body 1.

As can best be seen in Fig. 1d, at its back end the piston 2 features coupling means 4 for coupling the piston rod 5 in a detachable manner to the piston 2. These coupling means 4 comprise an axial cavity for receiving a corresponding coupling trunnion 6 of the piston rod 5 forming coupling means 6 of the piston rod 5 according to the claims.

As can be seen when comparing the Figures 1a and 1d showing the piston in the fully retracted position within the interior space of the container body 1 with the Figures 1b and 1c showing the piston in two other positions than the fully retracted position within said interior space, the coupling means 4 of the piston 2 further comprise two lugs 8 forming actuating elements according to the claims, which in the fully retracted position of the piston 2 radially expand into apertures 9 in the boundary walls of the interior space of the container body 1 (Figures 1a and 1d) and in all other positions of the piston 2 are tilted backwards by the boundary walls of the interior space, thereby forming two opposite protrusions 7 facing radially inwards from the boundary walls of the axial cavity of the coupling means 4 of the piston 2, and thus forming undercuts 7 according to the claims. When the piston again is positioned in the fully retracted position, the lugs 8 again expand into the apertures 9 in the boundary walls of the interior space of the container body 1 and by doing so, the protrusions 7 disappear, so that the axial cavity of the coupling means 4 of the piston 2 again shows a circular-cylindrical shape without undercuts.

Thus, the coupling means 4 of the piston 2 interact with the container body 1 in such a manner that, between the fully retracted position of the piston 2 and all other positions of the piston 2 within the interior space of the container body 1, the coupling means 4 of the piston 2 change their shape. In the fully retracted position of the piston 2, the receiving cavity for the piston rod 5 has an unrestricted circular-cylindrical shape while in all other positions of the piston 2 within the interior space this receiving cavity is restricted with two opposite protrusions 7 facing radially inwards from its boundary walls, thereby forming an undercut receiving cavity for the piston rod.

As can be seen when again comparing the Figures 1a and 1d with the Figures 1b and 1c, this functionality in combination with the especially adapted coupling trunnion 6 of the piston rod 5 leads to a situation where in the fully retracted position of the piston 2 (Figures 1a and 1d) a positive locking between the piston 2 and the piston rod 5 only exists in the pushing forward direction of the piston 2, so that the coupling trunnion 6 of the piston rod 5 without any substantial mechanical resistance can be inserted into or removed from the receiving cavity of the coupling means 4 of the piston, respectively, while in all other positions of the piston 2 within the interior space (Figures 1b and 1c) a positive locking between the piston 2 and the piston rod 5 exists in the pushing forward direction as well as in the retracting direction of the piston 2, so that the piston can be moved forward and backward in the interior space of the container body 1 by means of the piston rod 5 coupled to it.

In case the piston 2 starting from a not fully retracted position is retracted with the piston rod 5 into the fully retracted position, the positive locking situation in retracting direction between the piston 2 and the piston rod 5 is automatically terminated, as has been described into detail above, and in case after that the piston rod 5 is further moved into the retracting direction, it is automatically separated from the piston 2 which stays in the fully retracted position inside the interior space of the container body 1.

Due to the fact that for changing the shape of the coupling means 4 of the piston 2 the lugs 8 radially expand into apertures 9 in the boundary walls of the interior space of the container body 1, these lugs 8 in the fully retracted position of the piston 2 additionally establish a positive locking situation in retracting direction between the piston 2 and the container body 1 which in addition prevents any retracting of the piston 2 beyond the fully retracted position.

However, it is also envisaged to dispense with the before mentioned apertures 9 in the boundary walls of the interior space of the container body 1 and to allow a retraction of the piston 2 into a fully retracted position in which the lugs 8 axially leave the cylindrical interior space of the container body 1 and thereby radially expand and terminate the positive locking situation in retracting direction between the piston 2 and the piston rod 5. Also in this case, when the piston rod 5 is further moved into the retraction direction, it is reliably separated from the piston 2 without the risk of pulling the piston 2 out of the interior space of the container body 1.

While there are shown and described presently preferred embodiments of the invention, it is to be distinctly understood that the invention is not limited thereto but may be otherwise variously embodied and practiced within the scope of the following claims.

## Claims

1. Container for receiving a liquid, in particular a liquid drug, comprising a container body (1) with a cylindrical interior space defining a longitudinal axis (X) and with a piston (2) arranged in said interior space, which in a liquid-tight manner abuts to the boundary walls of interior space and is movable within said interior space along said longitudinal axis (X) in order to increase or reduce the volume of a fluid-tight space (3) defined by the boundary walls of interior space and by the front face of the piston (2),
wherein the piston (2) at its back side comprises coupling means (4) for coupling a piston rod (5) in a detachable manner to the piston (2) in order to be able to move the piston (2) forward and backward within the interior space of the container body (1),
and wherein the coupling means (4) of the piston (2) interact with the container body (1) in such a manner that, between a fully retracted position of the piston (2) and all other positions of the piston (2) within said interior space of the container body (1), said coupling means (4) of the piston (2) change their shape.

2. Container according to claim 1, wherein the coupling means (4) of the piston (2) comprise an axial cavity for receiving a coupling element (6) of the piston rod (5) to be coupled thereto, which cavity in all positions of the piston (2) except in the fully retracted position features one or several undercuts (7) which undercuts (7) are shortened or removed when the piston (2) is moved into the fully retracted position within said interior space of the container body (1).

3. Container according to one of the preceding claims, wherein the coupling means (4) of the piston (2) comprise an axial protrusion for being received in a cavity of an coupling element of a piston rod (5) to be coupled thereto, which protrusion in all positions of the piston (2) except in the fully retracted position features one or several undercuts which undercuts are shortened or removed when the piston (2) is moved into the fully retracted position within said interior space of the container body (1).

4. Container according to one of the preceding claims, wherein the coupling means (4) of the piston (2) comprise at least one axial or radial protrusion which in all positions of the piston (2) except in the fully retracted position is positioned in a first axial or radial position and when the piston (2) is moved into the fully retracted position, is moved into a second axial or radial position.

5. Container according to one of the preceding claims, wherein the coupling means (4) of the piston (2) comprises one or several actuating elements (8) which in the fully retracted position of the piston (2) radially expand and thereby effect the change in shape of the coupling means (4).

6. Container according to claim 5, wherein the boundary walls of the interior space of the container body (1) comprise one or several recesses or apertures (9) into which the actuating elements (8) expand when the piston (2) is moved into the fully retracted position within said interior space of the container body (1).

7. Container according to claim 5, wherein the cylindrical interior space of the container body (1) at its back end opens to the outside, in particular without any restriction which could stop a backward movement of the piston (2), and the actuating elements axially move out of the cylindrical interior space and radially expand when the piston (2) is moved into the fully retracted position.

8. Container according to one of the claims 5 to 7, wherein the actuating elements comprise one or several lugs (8), which radially expand when the piston (2) is brought into the fully retracted position.

9. Container according to one of the preceding claims, wherein the coupling means (4) of the piston (2) are made in one-piece with the body of the piston (2), and in particular, wherein the entire piston (2) including sealing elements and coupling means (4) is made as one-piece.

10. Container according to one of the preceding claims, wherein the container is a sterile container for medical use, in particular having a maximum volume with the piston (2) arranged in the fully retracted position of less than 50 ml, in particular less than 10 ml.

11. Container according to claim 10, wherein at the front end the container comprises a septum which can be pierced in order to get access to the fluid-tight space (3) defined by the boundary walls of interior space and by the front face of the piston (2).

12. Arrangement comprising a container according to one of the preceding claims and a piston rod (5) for moving the piston (2) of the container forward and backward within the interior space of the container body (1), wherein the piston rod (5) comprises coupling means (6) which are adapted to interact in the coupled state with the coupling means (4) of the piston (4) in such a manner that when the piston (2) is not in a fully retracted position, there exists a positive or non-positive locking between the piston (2) and the piston rod (5) in pushing forward direction as well as in retracting direction of the piston (2) and that when the piston (2) is retracted by means of the piston rod (5) into the fully retracted position, the positive or non-positive locking between the piston (2) and the piston rod (5) in retracting direction is terminated and upon a further movement of the piston rod (5) in said direction the piston rod (5) is separated from the piston (2) without further pulling the piston (2) in retracting direction.

13. Arrangement according to claim 12, wherein the piston rod (5) is part of an apparatus with drive means for moving the piston rod (5) forward and backward.

14. Method of operating the arrangement according to one of the claims 12 to 13, comprising the steps:
a) connecting the piston rod (5) and the piston (2) by coupling their coupling means (4, 6) with each other;
b) pushing the piston (2) by means of the piston rod (5) forward inside the interior space of the container body (1) thereby displacing air out of the fluid-tight space (3) defined by the boundary walls of interior space and by the front face of the piston (2);
c) retracting the piston (2) by means of the piston rod (5) inside the interior space of the container body (1) thereby receiving a liquid drug, in particular insulin, in the fluid-tight space (3) defined by the boundary walls of interior space and by the front face of the piston (2);
d) separating the piston (2) and the piston rod (5) by retracting the piston (2) by means of the piston rod (5) into the fully retracted position and subsequently moving the piston rod (5) further in retracting direction.

15. Method according to claim 14, wherein the steps b) and c) are successively performed more than once, thereby stepwise increasing the amount of liquid drug contained inside the fluid-tight space (3) of the container.

16. Method according to claim 15, wherein the steps b) and c) are successively performed until the fluid-tight space (3) of the container with the piston (2) arranged in the fully retracted position is substantially filled with the liquid drug.
